# EUROPÄISCHE PATENTSCHRIFT

(11) **EP 1 696 978 B1**
(45) Veröffentlichungstag und Bekanntmachung des Hinweises auf die Patenterteilung: **24.06.2015**
(21) Anmeldenummer: 04765014.8
(22) Anmeldetag: 07.09.2004
(51) Int. Cl.: A61F 2/82, A61L 31/14, A61L 31/18, A61L 27/04, A61L 27/58, A61F 2/06

(54) **RADIOOPAKER MARKER FÜR MEDIZINISCHE IMPLANTATE**
RADIO-OPAQUE MARKER FOR MEDICAL IMPLANTS
MARQUEUR RADIO-OPAQUE POUR IMPLANTS MEDICAUX

(30) Priorität: 24.12.2003 DE 10361942
(43) Veröffentlichungstag der Anmeldung: 06.09.2006
(73) Patentinhaber: Biotronik VI Patent AG, 6341 Baar (CH)
(72) Erfinder: GEROLD, Bodo, 91225 Zellingen (DE); HARDER, Claus, 91080 Uttenreuth (DE); HEUBLEIN, Bernd, deceased (DE); MÜLLER, Heinz, 91054 Erlangen (DE); HEUBLEIN, Eva, 30627 Hannover (DE); HEUBLEIN, Christoph, 30627 Hannover (DE); HEUBLEIN, Nora, 30627 Hannover (DE)
(74) Vertreter: Lindner-Vogt, Karin L.
(86) Internationale Anmeldenummer: PCT/EP2004/010081
(87) Internationale Veröffentlichungsnummer: WO 2005/065737

(56) Entgegenhaltungen:
- EP-A- 0 824 900
- EP-A- 0 966 979
- EP-A2- 0 894 503
- WO-A-99/03515
- WO-A-99/65537
- WO-A-2004/043474
- WO-A-2004/110515
- WO-A1-01/49340
- US-A1- 2002 040 239
- US-A1- 2002 138 136
- US-A1- 2003 199 993
- US-B1- 6 322 588
- US-B1- 6 423 085

## Beschreibung

Die Erfindung betrifft einen radioopaken Marker für medizinische Implantate und Implantate mit derartigen Markern.

In der modernen Medizintechnik werden in ständig zunehmenden Maße Implantate eingesetzt. Die Implantate dienen der Unterstützung von Gefäßen, Hohlorganen und Gangssystemen (endovaskuläre Implantate), zur Befestigung und temporären Fixierung von Gewebeimplantaten und Gewebetransplantationen, aber auch zu orthopädischen Zwecken, z.B. als Nagel, Platte oder Schraube. Häufig ist nur eine temporäre Stütz- bzw. Haltefunktion bis zum Abschluss des Heilungsprozesses oder Stabilisierung des Gewebes notwendig bzw. erwünscht. Um Komplikationen zu vermeiden, die aus dem dauerhaften Verbleib der Implantate im Körper resultieren, müssen die Implantate entweder wieder operativ entfernt werden oder sie bestehen aus einem Werkstoff, der allmählich im Körper abgebaut wird, d.h. biodegradierbar ist. Die Zahl biodegradierbarer Werkstoffe auf der Basis von Polymeren oder Legierungen ist stetig gewachsen, jedoch sind in vielen Anwendungsbereichen weiterhin auch die mechanischen Eigenschaften eines metallischen Werkstoffs unverzichtbar. In der Praxis haben sich bisher nur wenige metallische Werkstoffe als biodegradierbar bewährt. Zumeist werden Metalllegierungen aus Magnesium, Eisen und Wolfram vorgeschlagen.

Dokument EP 0894503 A2 offenbart einen Marker, bestehend aus einem biodegradierbaren Polymer als Basiskomponente, welches mit röntgensichtbaren Substanzen beschichtet ist oder in dem röntgensichtbaren Partikel inkorporiert sind.

Aus der EP 1 270 023 sind u.a. biodegradierbare Magnesiumlegierungen bekannt, die sich für endovaskuläre und orthopädische Implantate eignen. Die Legierungen können bis zu unter 5 Gew.% seltene Erden enthalten. Die meisten aus dem Stand der Technik bekannten biodegradierbaren Legierungen und Polymere für medizinische Implantate haben jedoch den Nachteil, dass sie nicht oder nur in einem nicht zufriedenstellenden Ausmaß in gängigen Röntgenverfahren erkennbar sind. Zur postoperativen Überwachung des Heilungsfortschrittes oder zur Kontrolle minimalinvasiver Eingriffe ist aber gerade die Röntgendiagnostik ein wichtiges Instrumentarium. So werden z.B. seit einigen Jahren Stents in den Koronararterien bei der akuten Myokardinfarkttherapie plaziert. Nach gängigen Verfahren wird ein Katheter, der den Stent in einem nichtexpandierten Zustand trägt, im Bereich der Läsion der koronaren Gefäßwand positioniert. Anschließend weitet sich der Stent entweder durch selbstexpansive Kräfte oder durch Inflation eines Ballons auf, um im expandierten Zustand eine Obstruktion der Gefäßwand zu verhindern. Der Vorgang der Positionierung und Expansion des Stents muss während der Prozedur durch den intervetionellen Kardiologen laufend überwacht werden.

Die Röntgensichtbarkeit eines aus einem metallischen oder polymeren Werkstoff hergestellten Implantats hängt zum einen von der Dicke des Materials und zum anderen von dessen linearen Schwächungskoeffizienten des Werkstoffs ab. Eisen als Bestandteil medizinischer Stähle hat beispielsweise einen linearen Schwächungskoeffizienten von 15,2 KeV/cm, was bei den filigralen Strukturen von Stents in der Regel schon nicht für ein kontrastreiches Überwachungsbild reicht. Bekannt ist ferner, dass der lineare Schwächungskoeffizient mit steigender Ordnungszahl im Periodensystem größer wird. So hat beispielsweise Gold einen linearen Schwächungskoeffizient von 101 KeV/cm.

Es ist daher bekannt, Implantate mit einer Beschichtung, einem Band oder eingearbeitetem oder sonst wie angeformten Marker zur Verbesserung der Röntgensichtbarkeit zu versehen. Für die Wahl des Markermaterials müssen im wesentlichen folgende Punkte beachtet werden:
- das Markermetall sollte nicht die mechanischen Eigenschaften des Implantats, insbesondere durch Erhöhung der Steifheit, verschlechtern
- der Marker muss biokompatibel sein und
- der Marker darf nicht während der Implantation, insbesondere bei Expansion oder Platzierung eines Stents, abblättern oder abplatzen.

Bekannte Markerverfahren sehen beispielsweise vor, Metallbänder aus Gold oder anderen Edelmetallen in bestimmten Bereichen des Stents anzubringen. Derartige Metallbänder können sich allerdings lösen, verschieben oder sogar abfallen. Ein nicht-degradierbarer aus einem Edelmetall Marker lässt die Bildung eines Lokalelements mit den meist unedleren Metallen des Grundköpers des Implantats erwarten, wodurch der Marker selbst sehr schnell aus der Struktur herausgelöst werden kann. Wenn der Marker aber sehr schnell abgelöst wird, kann das auch vor der vollständigen Endothelialisierung passieren; dann könnte der Marker fortgeschwemmt werden und zu einer Embolisation führen. Weiterhin besteht die Gefahr einer Abrasion der Intima während der Positionierung des Implantats.

Bei Metallbeschichtungsverfahren können durch chemische oder physikalische Vakuumabscheidungen (CVD oder PVD) radioopake Markierungsbereiche auf dem Implantat aufgebracht werden. Alternativ eignen sich Methoden wie ionenstrahlunterstützte Ablagerung (IBAD) und Mikrofusion, mit denen sehr homogene Beschichtungen im Mikrometerbereich auf der Implantatsoberfläche erzeugbar sind.

Die Aufbringung von Markerschichten und die Positionierung von Markerelementen auf den Implantaten aus biodegradierbaren Legierungen oder Polymeren ist alles andere als trivial und erfordert in der Regel eine individuelle Abstimmung der Werkstoffeigenschaften des Markerelements mit den weiteren im Implantat verwendeten Werkstoffen als auch Anpassungen im Design des Implantats. Bekannte Lösungsansätze lassen sich demnach nicht ohne weiteres auf neue Materialien, insbesondere die vielversprechenden biodegradierbaren Legierungen und Polymere, übertragen. Weiterhin sollte auch das Markerelement selbst weitestgehend biodegradierbar sein oder zumindest in physiologisch unbedenkliche Bestandteile überführt werden. Hierunter werden Bestandteile verstanden, die in Ihren Dimensionen deutlich kleiner sind, als die Abmessungen des Markers vor Implantation, die jedoch nicht weiter abgebaut werden und im Körper in unveränderter Form eingelagert werden.

Der vorliegenden Erfindung liegt demnach die Aufgabe zugrunde, einen zumindest teilweise biodegradierbaren Marker mit einer ausreichenden Röntgensichtbarkeit bereitzustellen.

Eine weitere Aufgabe der vorliegenden Erfindung ist es, geeignete Implantate für zumindest teilweise biodegradierbare Marker anzugeben.

Gemäß einem ersten Aspekt der Erfindung wird die gestellte Aufgabe gelöst durch einen radioopaken Marker für medizinische Implantate enthaltend
- 10 bis 90 Gewichtsprozente einer biodegradierbaren Basiskomponente,
- 10 bis 90 Gewichtsprozente eines oder mehrerer radioopaker Elemente aus der Gruppe I, Au, Ta, Y, Nb, Mo, Ru, Rh, Ba, La, Ce, Pr, Nd, Sm, Eu, Gd, Tb, Dy, Ho, Er, Tm, Yb, Lu, Hf, Ta, W, Re, Os, Ir und Bi als Markerkomponente und
- kleiner gleich 10 Gewichtsprozente an Restkomponenten,
wobei sich die genannten Komponenten zu 100 Gewichtsprozent ergänzen, dadurch gekennzeichnet, dass der Marker eine Legierung ist. Die Anteile der Komponenten sind dabei derart gewichtet, dass der Marker noch zumindest teilweise biodegradierbar ist, aber bei gängiger Dimensionierung des medizinischen Implantats dennoch eine ausreichende Röntgensichtbarkeit besteht.

Unter Biodegradierbarkeit im erfindungsgemäßem Sinne wird der über die Zeit durch chemische, thermische, oxidative, mechanische oder biologische Prozesse stattfindende zumindest teilweise Abbau des Markers im lebendem Organismus verstanden. Der Abbau betrifft zumindest die Basiskomponente des Markers. Die Markerkomponente ist entweder ihrerseits biodegradierbar oder sie liegt nach vollständiger Degradation der Basiskomponente als ein feinverteiltes Pulver vor, das problemlos aus dem Körper ausgeschieden oder ohne nennenswerte weitere biologische Interaktion in das Gewebe eingelagert wird. Mit anderen Worten, die möglicherweise verbleibenden nicht biodegradierbaren Bestandteile der Markerkomponente bilden keine in sich geschlossene Struktur, sondern zerfallen in kleinere Bestandteile. Gegebenenfalls ist daher die Markerkomponente in dieser Richtung durch in vitro Abbauexperimente zu evaluieren und anzupassen.

Eine bevorzugte Ausführungsform des Markers ist eine Legierung, die als Basiskomponente insbesondere eines oder mehrere biodegradierbare Elemente aus der Gruppe Magnesium, Eisen oder Zink enthält. Der Einsatz von Legierungen bietet vor allem den Vorteil, dass die Werkstoffeigenschaften der Legierungen im Vergleich zu herkömmlichen biodegradierbaren Legierungen, insbesondere Magnesiumlegierungen, einander deutlich angeglichen sind. Hierdurch wird die Herstellung von Implantaten mit Markerelementen vereinfacht, die aus einer Kombination herkömmlicher biodegradierbarer metallischer Legierungen als Grundkörper mit der erfindungsgemäßen radioopaken Legierung als Marker beruhen. Gerade an den Phasengrenzen zwischen Marker und Grundkörper des Implantats können die sonst häufig auftretenden Phasengrenzspannungen aufgrund der Angleichung der Werkstoffeigenschaften reduziert werden. Die Ausbildung von Lokalelementen bei heterogenen Legierungen zwischen den oft edleren Markerkomponenten und den unedleren Basiskomponenten stellt einen erwünschten, den Zerfall des Markers beschleunigenden Effekt dar.

Der Begriff 'Basiskomponente' im Sinne der Erfindung erfasst selbstverständlich auch jede Art von Kombination der genannten und weiterer zur Biodegradation geeigneter Werkstoffe. So kann die Basiskomponente ein Gemisch mehrerer biodegradierbarer Polymere und/oder Legierungen sein. Gemeinsames Merkmal all dieser denkbaren Basiskomponenten ist die zwingend notwendige gleichzeitige Anwesenheit einer von Markerkomponente.

Gemäß einer bevorzugten Ausführungsform der Erfindung, die auch in Kombination mit den zuvor genannten besonderen Ausführungsformen des Markers als Legierung oder Komposit ausführbar ist, umfasst die Markerkomponente eines oder mehrere Elemente aus der Gruppe I, Ta, Y, Ce, Nd, Sm, Gd, oder Dy. Die genannten Elemente zeichnen sich durch ihre hervorragende Biokompatibilität, günstigen linearen Schwächungskoeffizienten und gute Verfügbarkeit aus. Besonders bevorzugt ist insbesondere eine Markerkomponente, die ganz oder zumindest zu 90 Gewichtsprozent oder mehr an der Markerkomponente aus Tantal besteht. Tantal hat sich als besonders biokompatibel und leicht verarbeitbar erwiesen und kann insbesondere in Form kleinster Metallkügelchen mit wenigen µm Durchmesser Bestandteil des Markers sein. Zudem werden bei Einsatz von Y, Ce, Nd, Sm, Gd, oder Dy Magnesiumlegierungen hitzebeständiger und lassen sich besser verarbeiten. Die Elemente Y und Nd haben zudem in ersten Zellkulturuntersuchungen eine die Proliferation glatter humaner Muskelzellen hemmende Wirkung gezeigt, so dass der Einsatz dieser Elemente besonders sinnvoll im Zusammenhang mit endovaskulären Implantaten, wie zum Beispiel Koronarstents, erscheint.

Der Anteil der Basiskomponente am Marker liegt vorzugsweise bei 30 bis 70 Gewichtsprozenten, insbesondere bei 40 bis 60 Gewichtsprozenten. Hierdurch kann einerseits ein weitegehender Zerfall des Markers sichergestellt, aber andererseits eine noch gute Röntgensichtbarkeit sichergestellt werden.

Weiterhin ist bevorzugt, dass ein Anteil der Seltenerdmetalle einschließlich Yttrium als Bestandteile der Markerkomponente nicht mehr als 20 Gewichtsprozente, insbesondere nicht mehr als 15 Gewichtsprozente, am Marker beträgt. Hierdurch kann sichergestellt werden, dass die zumindest teilweise bestehende Toxizität bei höheren Dosen der genannten Metalle nicht zur Ausbildung von Nekrosen im umgebenden Gewebe führen dürfte.

Die Restkomponenten umfassen anorganische oder organische Füll-, Hilfs- oder Reststoffe, die ohne Einschränkungen der Funktionalität des Markers, z.B. aufgrund von Verunreinigungen der Ausgangsmaterialien, aber auch zur verbesserten Verarbeitung der einzelnen Komponenten vorhanden sind. Der Anteil der Restkomponenten am Marker liegt vorzugsweise bei ≤ 5 Gewichtprozenten, insbesondere ≤ 1 Gewichtsprozent. Denkbar ist, dass die Restkomponente ein pharmakologischer Wirkstoff ist, der z.B. die Gewebsverträglichkeit verbessert.

Gemäß einem zweiten Aspekt der Erfindung wird die gestellte Aufgabe durch ein selbst biodegradierbares Implantat gelöst, das zumindest einen Abschnitt enthält, der aus dem erfindungsgemäßen Marker besteht. Der Grundkörper des Implantats ist aus einem biodegradierbaren Werkstoff geformt, der beispielsweise auf Basis eines Polymers oder Metalls aufgebaut ist. Denkbar und besonders bevorzugt ist, dass der Grundkörper des Implantats ganz oder in Teilen aus dem Marker hergestellt. Alternativ kann der Grundkörper mit dem Marker beschichtet werden. Bei ersterer Variante sind Marker auf Basis einer Legierung bevorzugt, da diese häufig besser die mechanischen Anforderungen an den Werkstoff erfüllen. Ein Beispiel hierfür sind Koronarstents aus biodegradierbaren Magnesium-, Eisen- oder Wolframverbindungen. Im Falle einer Beschichtung mit dem Marker sind Beschichtungsdicken im Bereich von etwa 5 bis 100 µm bevorzugt, da diese eine ausreichende Röntgensichtbarkeit gewährleisten, jedoch noch nicht die Funktionalität des beschichteten Grundkörpers einzuschränken. Nach einer vorteilhaften Ausführung wird die Beschichtung nur endständig, z.B. durch Beschichtung mit einer Maske, auf das Implantat aufgebracht.

Vorzugsweise ist das Implantat zumindest bereichsweise aus einer aus dem Stand der Technik bekannten biodegradierbaren Magnesiumlegierung geformt. In diesem Falle wird als Marker eine Legierung bevorzugt, dessen Basiskomponente ebenfalls Magnesium ist. Hierdurch werden die Materialeigenschaften des Grundkörpers und des Markers einander angeglichen. Implantate aus bekannten Magnesiumlegierungen lassen sich daher besonders gut mit den erfindungsgemäßen Legierungen des Markers beschichten oder in angeformten Teilbereichen ausbilden. Die Materialähnlichkeit erhöht die Haftung zwischen den verschiedenen Legierungen, so dass ein Abblättern oder Abplatzen der Beschichtung oder ein Bruch entlang der Phasengrenzen zwischen der Markerlegierung und bekannten Magnesiumlegierungen des Grundkörpers bei mechanischer Belastung vermieden wird. Aufgrund der sehr ähnlichen Eigenschaften ist es zum Beispiel im allgemeinen nicht notwendig ein Stentdesign grundlegend für die Auf- oder Einbringung der Markerlegierung zu überarbeiten.

Das zumindest weitestgehend biodegradierbare Implantat ist vorzugsweise ein endovaskuläres Implantat, insbesondere ein Stent oder ein Okkluder. Ferner findet der erfindungsgemäße Marker, insbesondere in Form einer Legierung, vorzugsweise Verwendung in orthopädischen Implantaten, wie Nägeln, Schrauben, Clips oder alloplastischen Prothesen, wie z.B. Anastomose-Implantate in Form eines kleinen Röhrchen zum Verbinden zweier Gefäßenden.

Die Erfindung wird nachfolgend in Ausführungsbeispielen näher erläutert.

Ein Stent herkömmlicher Bauart mit einem Grundgerüst aus der biodegradierbaren Magnesiumlegierung WE43 (Zusammensetzung: Y 4,1 Gew.%; Nd 2,2 Gew.%; Zr 0,5 Gew.%; andere < 0,4 Gew.% und Rest Mg) wird nachfolgend auf verschiedene Weise mit einem Marker verbunden.

Nach einer ersten Variante besteht der Marker aus einer durch PVD aus einem Mg/Y-Target abgeschiedenen Beschichtung. Das Mg/Y-Target hat beispielsweise Zusammensetzung von etwa 85 Gew.% Magnesium und 15 Gew.% Yttrium. Selbstverständlich können die Elemente für die Basis- und Markerkomponenten je nach gewünschter Zusammensetzung des abzuscheidenden Markers variiert werden. Derartige Abscheidungsverfahren sind dem Fachmann hinlänglich bekannt, so dass auf ein näheres Eingehen verzichtet wird. Das Abscheidungsverfahren wird dabei so gesteuert, dass eine etwa 5 bis 100 µm dicke Schicht des Markers entsteht. Sofern gewünscht, kann der Abscheidungsort durch Fokussierung des Materialstrahls oder Maskierung örtlich begrenzt werden, z.B. zur Herstellung eines endständigen zirkulären Markers.

Gemäß einer alternativen Variante kann die Oberfläche des Implantats mit einem Komposit aus einer biodegradierbaren polymeren Basiskomponente und einer Markerkomponente bedeckt werden. Dies kann beispielsweise derart erfolgen, dass als Markerkomponenten agierende winzige Tantal- oder Gadoliniumkügelchen einem als Basiskomponenten agierenden Polymer auf Grundlage von polymerisierter Hyaluronsäure beigesetzt werden. Die entstehende Dispersion wird durch gängige Tauch- oder Sprühverfahren anschließend auf das Implantat aufgebracht und getrocknet. Ein Gewicht der trockenen polymerisierten Hyaluronsäure zum Gewicht der Tantal- oder Gadoliniumkügelchen liegt beispielsweise bei etwa 50 zu 50. Der Marker kann örtlich begrenzt, z. B. in dafür vorgesehene Kavitäten im Grundkörper des Implantats eingebracht werden, oder als Beschichtung das gesamte Implantat bedecken.

## Patentansprüche

1. Radioopaker Marker für medizinische Implantate enthaltend
- 10 bis 90 Gewichtsprozente einer biodegradierbaren Basiskomponente,
- 10 bis 90 Gewichtsprozente eines oder mehrerer radioopaker Elemente aus der Gruppe I, Au, Ta, Y, Nb, Mo, Ru, Rh, Ba, La, Ce, Pr, Nd, Sm, Eu, Gd, Tb, Dy, Ho, Er, Tm, Yb, Lu, Hf, W, Re, Os, Ir und Bi als Markerkomponente und
- kleiner gleich 10 Gewichtsprozente an Restkomponenten,
wobei sich die genannten Komponenten zu 100 Gewichtsprozenten ergänzen, **dadurch gekennzeichnet, dass** der Marker eine Legierung ist.

2. Marker nach Anspruch 1, **dadurch gekennzeichnet, dass** die Legierung als Basiskomponente eines oder mehrere biodegradierbare Elemente aus der Gruppe Magnesium, Eisen oder Zink enthält.

3. Marker nach einem der vorhergehenden Ansprüche, **dadurch gekennzeichnet, dass** die Markerkomponente eines oder mehrere Elemente aus der Gruppe I, Ta, Y, Ce, Nd, Sm, Gd, oder Dy umfasst.

4. Marker nach Anspruch 3, **dadurch gekennzeichnet, dass** die Markerkomponente ganz oder zumindest zu 90 Gewichtsprozent oder mehr an der Markerkomponente aus Tantal besteht.

5. Marker nach einem der vorhergehenden Ansprüche, **dadurch gekennzeichnet, dass** der Anteil der Basiskomponente am Marker bei 30 bis 70 Gewichtsprozenten, insbesondere bei 40 bis 60 Gewichtsprozenten, liegt.

6. Marker nach einem der vorhergehenden Ansprüche, **dadurch gekennzeichnet, dass** ein Anteil der Seltenerden und von Yttrium als Bestandteile der Markerkomponente nicht mehr als 20 Gewichtsprozente, insbesondere nicht mehr als 15 Gewichtsprozente, am Marker beträgt.

7. Marker nach einem der vorhergehenden Ansprüche, **dadurch gekennzeichnet, dass** der Anteil der Restkomponente am Marker bei ≤ 5 Gewichtprozenten, insbesondere ≤ 1 Gewichtsprozent, liegt.

8. Biodegradierbares Implantat mit einem Abschnitt oder einer Beschichtung aus einem Marker nach einem oder mehreren der Ansprüche 1 bis 7.

9. Biodegradierbares Implantat mit einem ganz oder in Teilen aus einem Marker nach einem oder mehreren der Ansprüche 1 bis 7 bestehenden Grundkörper.

10. Biodegradierbares Implantat nach den Ansprüchen 8 oder 9, **dadurch gekennzeichnet, dass** der Grundkörper aus einer biodegradierbaren Magnesiumlegierung geformt ist.

11. Biodegradierbares Implantat nach einem der Ansprüche 8 bis 10, **dadurch gekennzeichnet, dass** das Implantat ein endovaskuläres Implantat, ein Okkluder, ein orthopädisches Implantat oder eine allosplastische Prothese ist.

## Claims

1. A radiopaque marker for medical implants comprising:
- 10 to 90 % by weight of a biodegradable base component,
- 10 to 90 % by weight of one or more radiopaque elements selected from the group consisting of I, Au, Ta, Y, Nb, Mo, Ru, Rh, Ba, La, Ce, Pr, Nd, Sm, Eu, Gd, Tb, Dy, Ho, Er, Tm, Yb, Lu, Hf, W, Re, Os, Ir, and Bi as a marker component, and,
- less than or equal to 10 % by weight of residual components,
the components cited adding up to 100 % by weight, **characterised in that** the marker is an alloy.

2. The marker according to Claim 1, **characterised in that** the marker contains one or more biodegradable elements selected from the group consisting of magnesium, iron, or zinc as the base component.

3. The marker according to one of the preceding claims, **characterised in that** the marker component comprises one or more elements selected from the group consisting of I, Ta, Y, Ce, Nd, Sm, Gd, or Dy.

4. The marker according to Claim 3, **characterised in that** the marker component comprises tantalum entirely or to an extent of at least 90 % by weight or more of the marker component.

5. The marker according to one of the preceding claims, **characterised in that** the proportion of the base component in the marker is 30 to 70 % by weight, in particular 40 to 60 % by weight.

6. The marker according to one of the preceding claims, **characterised in that** a proportion of the rare earths and of yttrium as components of the marker component is not more than 20 % by weight, in particular not more than 15 % by weight, in the marker.

7. The marker according to one of the preceding claims, **characterised in that** the proportion of the residual component in the marker is ≤ 5 % by weight, in particular ≤ 1 % by weight.

8. A biodegradable implant having a section or a coating formed of a marker according to one of more of Claims 1 to 7.

9. A biodegradable implant comprising a main body consisting wholly or in parts of a marker according to one or more of Claims 1 to 7.

10. The biodegradable implant according to Claims 8 or 9, **characterised in that** the main body is formed from a biodegradable magnesium alloy.

11. The biodegradable implant according to one of Claims 8 to 10, **characterised in that** the implant is an endovascular implant, an occluder, an orthopaedic implant, or an alloplastic prosthesis.

## Revendications

1. Marqueur radio-opaque pour implants médicaux contenant
- 10 à 90 pourcents en poids d'une composante de base biodégradable,
- 10 à 90 pourcents en poids d'un ou de plusieurs éléments radio-opaques faisant partie du groupe rassemblant I, Au, Ta, Y, Nb, Mo, Ru, Rh, Ba, La, Ce, Pr, Nd, Sm, Eu, Gd, Tb, Dy, Ho, Er, Tm, Yb, Lu, Hf, W, Re, Os, Ir et Bi en tant que composante de marquage, et
- un pourcentage inférieur à 10 pourcents en poids de composantes résiduelles,
où lesdites composantes se complètent à 100 pourcents en poids,
**caractérisé en ce que** le marqueur est un alliage.

2. Marqueur selon la revendication 1, **caractérisé en ce que** l'alliage servant de composante de base contient un ou plusieurs éléments biodégradables faisant partie du groupe constitué par le magnésium, le fer ou le zinc.

3. Marqueur selon l'une des revendications précédentes, **caractérisé en ce que** la composante de marquage comprend un ou plusieurs éléments faisant partie du groupe rassemblant I, Ta, Y, Ce, Nd, Sm, Gd ou Dy.

4. Marqueur selon la revendication 3, **caractérisé en ce que** la composante de marquage est constituée de tantale totalement ou au moins à 90 pourcents en poids, ou plus de la composante de marquage.

5. Marqueur selon l'une des revendications précédentes, **caractérisé en ce que** la proportion de la composante de base dans le marqueur se situe entre 30 et 70 pourcents en poids, notamment entre 40 et 60 pourcents en poids.

6. Marqueur selon l'une des revendications précédentes, **caractérisé en ce que** la proportion de terres rares et d'yttrium en tant que constituants de la composante de marquage ne dépasse pas 20 pourcents en poids, notamment pas 15 pourcents en poids, dans le marqueur.

7. Marqueur selon l'une des revendications précédentes, **caractérisé en ce que** la proportion de la composante résiduelle dans le marqueur se situe à une valeur ≤ à 5 pourcents en poids, notamment ≤ 1 pourcent en poids.

8. Implant biodégradable avec une partie ou un revêtement constitué d'un marqueur selon l'une ou plusieurs parmi les revendications 1 à 7.

9. Implant biodégradable avec un corps de base constitué entièrement ou en parties par un marqueur selon l'une ou plusieurs parmi les revendications 1 à 7.

10. Implant biodégradable selon les revendications 8 ou 9, **caractérisé en ce que** le corps de base est fait dans un alliage de magnésium biodégradable.

11. Implant biodégradable selon l'une des revendications 8 à 10, **caractérisé en ce que** l'implant est un implant endovasculaire, un occludeur, un implant orthopédique ou une prothèse alloplastique.
